# EUROPEAN PATENT SPECIFICATION

(11) **EP 0 472 664 B1**
(45) Date of publication and mention of the grant of the patent: **17.03.1999**
(21) Application number: 90909914.5
(22) Date of filing: 21.05.1990
(51) Int. Cl.: A61M 16/00

(54) **INSPIRATORY AIRWAY PRESSURE SYSTEM**
DRUCKSYSTEM FÜR ATMUNGSWEGE
SYSTEME DE PRESSION POUR VOIES INSPIRATOIRES

(30) Priority: 19.05.1989 US 354143; 24.04.1990 US 513757
(43) Date of publication of application: 04.03.1992
(60) Divisional application: 98115332.3
(73) Proprietor: PURITAN-BENNETT CORPORATION, Lenexa, KS 66215-2198 (US)
(72) Inventor: TRIMBLE, Russell, L., Overland Park, KS 66213 (US); GRUENKE, Roger, A., Overland Park, KS 66212 (US); SNOOK, James, A., Overland Park, KS 66212 (US); ORLT, Jiri, G., Lenexa, KS 66215 (US); LOETHEN, Steven, W., Independence, MO 64057 (US)
(74) Representative: Belcher, Simon James
(86) International application number: US9002800
(87) International publication number: WO9014121

(56) References cited:
- WO-A-82/03326
- WO-A-88/10108
- US-A- 3 028 873
- US-A- 3 741 208
- US-A- 3 840 006
- US-A- 3 863 630
- US-A- 3 961 627
- US-A- 3 972 327
- US-A- 4 156 426
- US-A- 4 239 039
- US-A- 4 357 936
- US-A- 4 508 117
- US-A- 4 527 557
- US-A- 4 570 631
- US-A- 4 637 385
- US-A- 4 667 669
- US-A- 4 686 974
- US-A- 4 726 366
- US-A- 4 776 333
- US-A- 4 782 832
- US-A- 4 802 485
- US-A- 4 807 616
- US-A- 4 823 788
- US-A- 4 838 258
- US-A- 4 883 051
- US-A- 4 915 103
- US-A- 4 938 212

## Description

The present invention relates to continuous positive airway pressure breathing apparatus for treating sleep apnea according to the preamble of claim 1. More particularly, the present invention is concerned with an apparatus and method for imposing a positive pressure on the patient's airways just prior to the onset of inhalation in order to induce and/or permit inhalation, and for subsequently reducing the pressure on the airways to ease exhalation effort.

Obstructive sleep apnea is a sleep disorder characterized by relaxation of the airway including the genioglossus throat muscle tissue during sleep. When this occurs, the relaxed muscle can partially or completely block the patient's airway, a condition more prevalent in overweight patients. Partial blockage can result in snoring. Complete blockage can result in sleep apnea.

When complete blockage occurs, the patient's inhalation efforts do not result in the intake of air and the patient becomes oxygen deprived. In reaction, the patient begins to awaken. Upon reaching a nearly awakened state, the genioglossus muscle resumes normal tension which clears the airway and allows inhalation to occur. The patient then falls back to a deeper sleep whereupon the genioglossus muscle again relaxes and the apneic cycle repeats.

Central apnea is when no inspiratory effort occurs or is delayed. Central apnea may be combined with obstructive apnea, known as mixed apnea. Other breathing irregularities such as Cheynes Stockes breathing may have apneic intervals when intake airflow ceases.

In some patients, sleep apnea events can occur dozens of times during the course of a sleep session. In consequence, the patient never achieves a fully relaxed, deep sleep session because of the repetitive arousal to a nearly awakened state. The patient is also deprived of REM (rapid eye movement) sleep. People afflicted with sleep apnea are continually tired even after an apparently normal night's sleep.

In order to treat obstructive sleep apnea, the so-called continuous positive airway pressure (CPAP) system has been devised in which a prescribed level of positive airway pressure is continuously imposed on the patient's airways. The presence of such positive pressure on the airways provides a pressure splint to offset the negative inspiratory pressure to maintain tissue position tension and thereby maintain an open patient airway. The positive airway connection with a patient is typically achieved by way of a nasal pillow such as that disclosed in US-4782832 in which the nasal pillow seals with the patient's nares and imposes the positive airway pressure on the nasal passages.

The CPAP system meets with objections from patients, however, because the patient must exhale against the positive pressure. This increases the work to exhale. Some patients have difficulty getting used to this and as a result, may discontinue the therapy. Drying of the nose and airway due to continuous circulation of room air is also a complaint. Also, exhaled carbon diode tends to remain in some nasal masks with CPAP therapy.

In prescribing CPAP therapy, it is usually necessary for a patient to spend one or two nights in a sleep treatment laboratory where it is first determined whether the patient has a respiratory disorder such as sleep apnea. If so, the patient is then fitted with a CPAP device whereupon the required gas pressure is determined for providing the necessary air splint to maintain airway patency.

The required pressure for maintaining patency is usually higher when the patient is sleeping on his or her back than when sleeping in a side rest position. The higher pressure is usually prescribed in order to ensure sufficient pressure in all sleeping positions. The higher pressure is not needed, however, in all circumstances. For example, before the patient has fallen asleep and in the early stages of sleep, the higher pressures are not needed. Additionally, the higher pressures are often not needed during deep sleep when the patient is in the side rest position. Furthermore, a given patient may only be subject to sleep apnea under certain conditions such as when the patient is extremely tired or under the influence of alcohol or sleep-inducing drugs. As a result, the patient is subjected to the discomfort of the high prescription pressures even when not needed.

WO-A-88/10108 discloses a continuous positive airway pressure breathing apparatus for treating sleep apnea which comprises a blower for supplying breathing gas which can supply gas to a patient at a positive pressure throughout the patient's breathing cycle, and means for controlling the supply of breathing gas in response to sounds that are detected from the patient.

The inspiratory airway pressure apparatus defined in claim 1 solves the prior art problems as outlined above. More particularly, the preferred system hereof initiates inspiratory nasal air pressure just prior to inhalation in order to provide a pressure splint to offset negative inspiratory pressure and retain the normal position of the genioglossus muscle thereby ensuring an open patient airway, and subsequently reduces the pressure for ease of exhalation. Airflow during this exhalation is primarily the patient's exhalent with desirable humidity.

The preferred apparatus is adapted for connection with a patient-coupled gas delivery device for pressurizing at least a portion of a patient's respiratory passages, such as the nasal passages, with a breathable gas, preferably ambient air which may be supplemented with oxygen, at a controllable gas pressure. The apparatus includes means for determining a point in the patient's breathing cycle before the onset of an inhalation phase and subsequent to a prior inhalation phase, and further includes gas control means for initiating, at the determined point in the breathing cycle, an increase in the gas pressure toward a selected, and preferably prescribed, high pressure level. `The gas control means further controls the gas pressure at the higher level during at least a portion of the inhalation phase and subsequently lowers the gas pressure in order to present a lower pressure level during at least a portion of the subsequent exhalation phase.

In preferred forms, the apparatus tracks the patient's breathing cycle, thereby determines the end of the exhalation phase of the breathing cycle, and initiates the pressure increase at that point in the breathing cycle. Alternatively, the apparatus determines an interval time as the point in the breathing cycle for increasing the inspiratory pressure as a function of previous breath rates and inhalation and exhalation intervals.

The apparatus desirably includes a controllable, variable speed blower for supplying ambient air above atmospheric pressure, a nasal pillow for coupling with the patient's nares, a conduit intercoupling the blower and nasal pillow, and a controllable, variably positionable vent valve coupled with the conduit for venting air therefrom. The preferred apparatus also includes a controller operably coupled with the blower and with the vent valve, and a pressure transducer for sensing the patient's nasal air pressure.

In operation, the controller maintains a set point pressure by varying the position of the vent valve to vent greater or lesser amounts of air from the conduit in correspondence with patient exhalation and inhalation. The controller further tracks the position of the vent valve and thereby tracks the patient's breathing cycle. That is to say, as the patient inhales during the inhalation cycle, the vent valve must close partially to maintain the pressure of the ambient air as the patient inhales. In this way, the movement of the valve corresponds to the inhalation of the patient. Similarly, during exhalation at a preferred lower pressure set point, the vent valve must vent greater amounts of ambient air from the conduit which tracks the patient's exhalation phase. By such tracking, even at different set point pressures, the system hereof is able to increase the set point pressure predictably prior to the onset of inhalation, and to subsequently decrease the pressure during the next exhalation phase.

### Brief Description of the Drawing Figures

Figure 1 is a plan view of the head of a sleeping patient shown wearing the preferred patient-coupling head gear for use with the present invention;
Fig. 2 is a side elevational view of the patient's head and head gear of Fig. 1 shown coupled with the preferred housing cabinet of the dual conduit embodiment of the present invention;
Fig. 3 is a schematic representation of the single-conduit embodiment of the present invention;
Fig. 4 is a schematic representation of the dual-conduit embodiment of Fig. 2;
Fig. 5 is an elevational view of the preferred vent valve element in position over the vent ends of the dual-conduit embodiment of Fig. 4;
Fig. 6 presents graphical illustrations of a typical breathing cycle including an inhalation phase and an exhalation phase, of the nasal air pressure imposed on the patient's airway during the breathing cycle, and of the vent valve steps required to maintain the set point pressures;
Fig. 7 is an electrical schematic illustration of the microcontroller and associated components of the present invention;
Fig. 8 is an electrical schematic of the blower motor control;
Fig. 9 is an electrical schematic of the stepper motor control for the vent valve;
Fig. 10 is a schematic illustration of a pressure transducer circuit;
Fig. 11 is a computer program flowchart illustrating the START-UP portion of the main routine;
Fig. 12 is a computer program flowchart of the MAIN LOOP portion of the main routine;
Fig. 13 is a computer program flowchart of the VALVE STEP subroutine;
Fig. 14 is a computer program flowchart of the ADC interrupt;
Fig. 15 is a computer program flowchart of the CHECK BLOWER SPEED subroutine;

### Detailed Description of the Preferred Embodiments

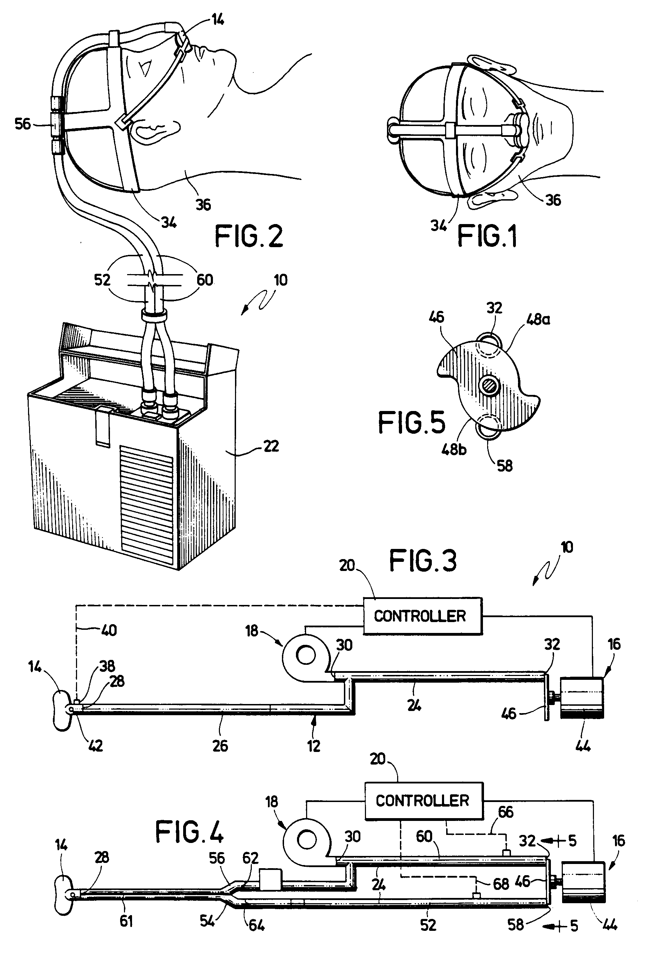

With reference to the drawing figures, Fig. 3 schematically illustrates the single conduit embodiment of the preferred inspiratory airway pressure apparatus 10 which broadly includes an elongated, flexible, hose or conduit 12, nasal pillow 14 connected to one end of conduit 12, vent valve assembly 16 positioned adjacent the opposed, open, vent end of conduit 12, blower unit 18 fluidically coupled with conduit 12 between pillow 14 and vent valve assembly 16, and controller 20 which is adapted for pneumatic connection with nasal pillow 14 and electrical connection with vent valve assembly 16 and blower unit 18.

In the preferred embodiment, vent valve assembly 16, blower unit 18, and controller 20 are housed within cabinet 22 such as that illustrated in Fig. 2 in connection with the dual-conduit embodiment. In this regard, conduit 12 presents an interior portion which is housed within cabinet 22 and exterior portion 26 which extends from the cabinet to nasal pillow 14. Conduit 12 additionally presents coupling end 28 coupled to nasal pillow 14, inlet end 30 coupled with blower unit 18 for receiving a supply of breathable gas, preferably ambient air therefrom, and vent end 32 positioned adjacent vent valve assembly 16.

Nasal pillow 14 is the preferred patient-coupling device and is further illustrated in US-4782832. Head gear 34 holds nasal pillow 14 on the head of patient 36 in order to fluidically couple with the respiratory passages of patient 36, and preferably with the patient's nares. Nasal pillow 14 is configured to present pressure sensor fitting 38 which is coupled with controller 20 by pneumatic line 40 which is preferably routed within conduit 12 so that line 40 is conveniently out of the way and less likely to be pinched or restricted by the patient during use of apparatus 10. Nasal pillow 14 also includes vent port 42 defined therethrough which continuously vents a small amount of pressure from nasal pillow 14 in order to prevent moisture buildup and subsequent condensation therein. Port 42 also prevents build up of exhaled gases including carbon dioxide.

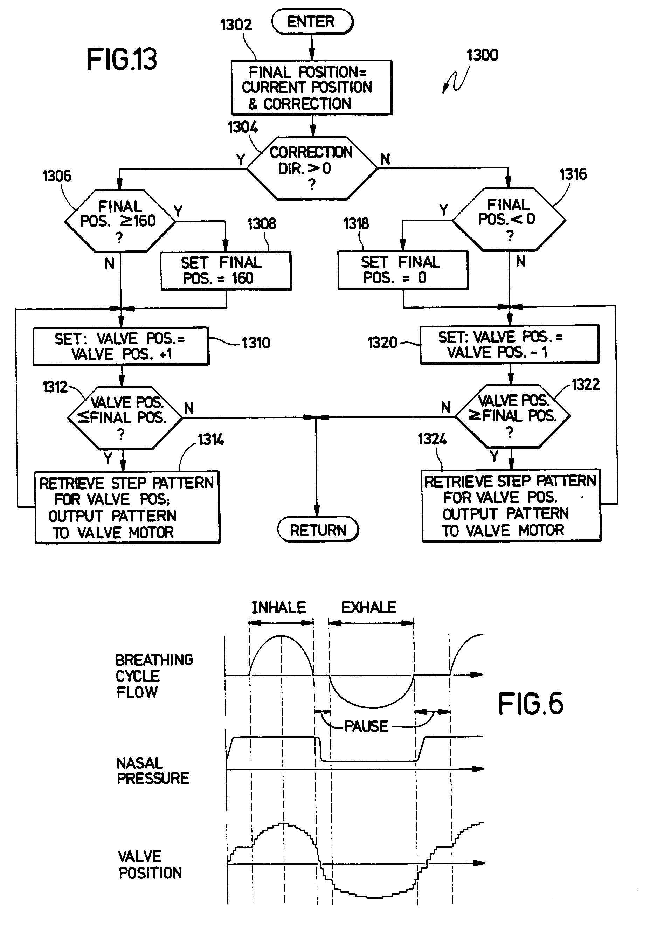

Vent valve assembly 16 includes stepper motor 44 and valve element 46 connected to the output shaft thereof. Valve element 46 is preferably constructed of a flat plate configured to present two, opposed, arcuate, cam-like edges 48a,b as illustrated in Fig. 5. Element 46 is positioned adjacent vent end 32 of conduit 12 so that as stepper motor 44 rotates valve element 46 in a clockwise direction as viewed in Fig. 5, edge 48a progressively covers and thereby restricts vent end 32. Conversely, as motor 44 rotates element 46 in a counterclockwise direction, edge 48a progressively exposes an increasing area of vent end 32 to vent additionally gas therefrom.

Fig. 4 illustrates the dual-conduit second embodiment of preferred apparatus 10. This embodiment is similar to that of Fig. 3 and corresponding components are numbered the same. Second embodiment 50 additionally includes exhaust hose 52 presenting connection end 54 fluidically coupled to conduit exterior portion 26 at junction 56, and presents exhaust end 58 positioned adjacent valve element 46 in the same opening/closing relationship with arcuate edge 48b as vent end 32 presents to arcuate edge 48a. With this configuration, conduit 12 additionally presents inhalation hose 60 between juncture 56 and blower unit 18. In the dual hose model, nasal pillow 14 does not include vent hole 42, and the tube between ends 54 and 28 include divider 61 to separate it into two separate passages. Second embodiment 50 may also include inhalation check valve 62 disposed within inhalation hose 60 adjacent juncture 56, and exhalation check valve 64 disposed within exhaust hose 52 also adjacent juncture 56. Inhalation check valve 62 prevents passage of patient exhalation therethrough toward vent end 32 and thereby requires that the patient's exhalation exit the system through exhaust end 58. Pneumatic lines 66 and 68 respectively couple controller 20 with inhalation hose 60 and exhaust hose 52.

By way of overview, controller 20 controls apparatus 10 in order to increase the gas pressure presented to the patient at a time in the patient's breathing cycle just prior to inhalation, and to subsequently lower the pressure for ease of exhalation. `The upper graph of Fig. 6 illustrates a typical breath cycle air flow. During inhalation, the flow rate of gas to the patient gradually increases to a maximum and then decreases. At the end of inhalation, the patient typically experiences a slight pause before exhalation begins. During exhalation, the exhaled gas flow from the patient gradually increases to a maximum and then decreases again. A post-exhalation pause, typically somewhat longer than the post-inhalation pause, follows exhalation. After the post-exhalation pause, the patient again then begins inhalation.

The middle graph of Fig. 6 illustrates the nasal airway pressure presented to patient 36 during operation of apparatus 10. With patients subject to sleep apnea, it Is desirable to increase nasal airway pressure just prior to inhalation to splint airway pressure in order to position genioglossus tissue and thereby maintain the airway open. Accordingly, this middle graph illustrates an increase in the nasal airway pressure just prior to inhalation to a selected prescription pressure level sufficient to push surrounding tissue aside and open this airway. After completion of inhalation, the set point pressure presented to the nasal airway Is reduced so that exhalation occurs against a low or even zero pressure level relative to ambient. At the end of exhalation, the nasal airway pressure is again increased prior to the next inhalation phase.

To accomplish these pressure variations, blower unit 18, in one embodiment of the invention, produces a generally constant volume per unit time of breathable gas which is selectively vented through vent end 32. The vented gas volume is controlled by vent valve assembly 16.

The bottom graph of Fig. 6 graphically depicts the various positions of valve element 46 in relation to vent end 32 in order to achieve the desired nasal airway pressure profile illustrated in the middle graph. For example, during the post-exhalation pause, controller 20 activates stepper motor 44 to rotate valve element 46 in a clockwise direction (as viewed in Fig. 5) in order to increase the nasal airway pressure to the desired set point as sensed by controller 20 by way of pneumatic line 40. When the patient begins to inhale, gas output from blower unit 18 is inhaled by the patient. In order to maintain the set point pressure, the controller then rotates valve element 46 in stepwise fashion further in the clockwise direction to reduce the amount of gas being vented. As inhalation passes its peak flow rate, controller 20 begins to reverse the position of valve element 46 to vent additional gas for maintaining the set point pressure.

At the end of inhalation, a lower pressure set point is desired and controller 20 continues, in stepwise fashion, to rotate valve element 46 in the counterclockwise direction to vent additional amounts of gas for achieving a new lower set point pressure.

At the end of the post-inhalation pause, the patient begins to exhale. In order to maintain desired lower set point pressure, the additionally exhausted gas from the patient must be vented through vent end 32. Accordingly, controller 20 causes valve element 46 to further rotate in a clockwise direction to open vent end 32 even further. As the exhalation flow rate decreases, controller 20 rotates valve element 46 in a clockwise direction to decrease venting in order to maintain the lower set point pressure. At the end of exhalation, controller 20 then causes valve element 46 to rotate further in the clockwise direction to increase the pressure to the higher pressure set point. This induces tension in the genioglossus muscle to open the airway in preparation for the next inhalation phase.

Inspection of the upper and lower graphs reveals a similarity in the profile of the curves. That is to say, controller 20 is able to track a patients breathing cycle by tracking the stepped positions of valve element 46 required to maintain the set point pressures. In this way, controller 20 is able to determine the end of respective inhalation/exhalation phases and to predict exhalation and inhalation interval times.

Turning now to controller 20, it provides electrical outputs to control the speed of blower unit 18 and the position of stepper motor 44. Controller 20 receives electrical feedback from blower unit 18 indicative of the speed thereof, and a pneumatic input by way of pneumatic line 40 to indicate the pressure at nasal pillow 14 and thereby in the patient's nasal airway passages.

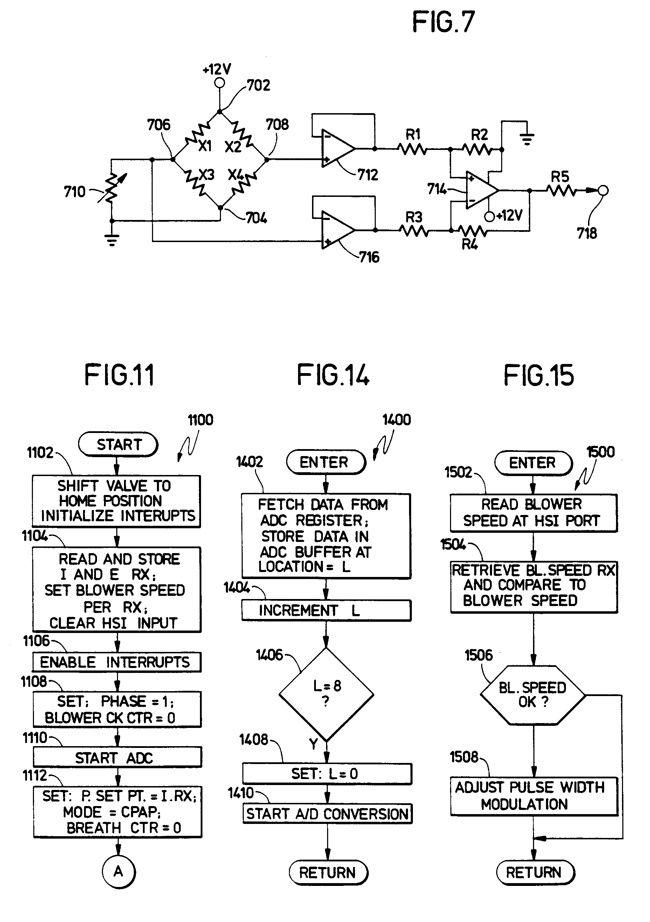

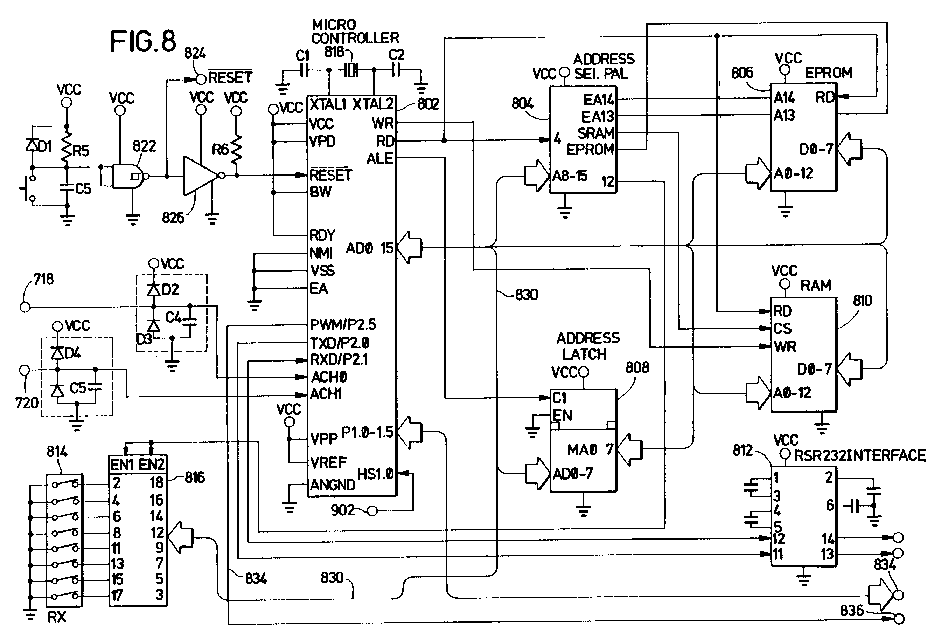

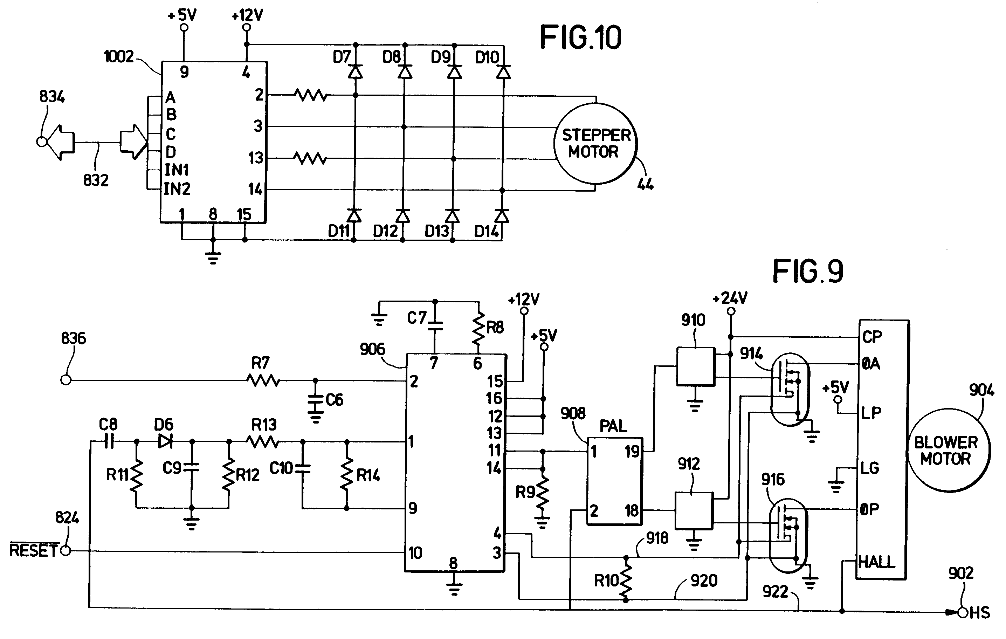

Controller 20 includes pressure transducer circuit 700 (Fig. 7) for providing an electrical input indicative of the pressure at nasal pillow 14 to microcontroller circuit 800 (Fig. 8) which in turn provides outputs to blower motor circuit 900 (Fig. 9) and stepper motor circuit 1000 (Fig. 10). Additionally, controller 20 includes a conventional 120 v.a.c. to +5 v.d.c., +12 v.d.c., and +24 v.d.c. power supply (not shown) suitable for digital and analog, solid state integrated circuit components.

Pressure transducer circuit 700 illustrated in Fig. 7 is typical of the pressure transducer circuit for both the single and dual conduit embodiments of the present invention. That is to say, the single conduit embodiment of Fig. 3 uses only one pressure transducer whereas the embodiment schematically illustrated in Fig. 4 uses two pressure transducers both using a circuit as illustrated in Fig. 7.

The preferred pressure transducer includes SENSYM type SX01DN having a zero-to 70-cm. water operational range. The preferred transducer includes four strain gages arranged in a conventional Wheatstone bridge 701 having strain gages X1, X2, X3, and X4 presenting a nominal 4650 ohms each. Bridge 701 presents excitation terminal 702 connected to +12 v.d.c. and an opposed excitation terminal 704 connected to ground as shown. Bridge 701 produces outputs at terminals 706 and 708. Zero adjustment potentiometer 710 interconnects terminals 704 and 706.

The output from terminal 708 is connected to the positive input terminal of operational amplifier 712 (one-half of Type LT1014). The output of operational amplifier 712 provides feedback to the negative input terminal thereof, and, by way of reststor R1 (1K ohms) supplies the positive input terminal of amplifier 714. The output is also connected to ground by way of resistor R2 (750K ohms).

Strain gage bridge output terminal 706 is connected to the positive input terminal of operational amplifier 716 (the other half of unit LT1014). The output from amplifier 716 provides feedback to the negative input terminal thereof and is connected by way of resistor R3 (1K ohms) to the negative input terminal of amplifier 714.

The output from amplifier 714 provides feedback to the negative input terminal thereof by way of resistor R4 (750K ohms). The output from amplifier 714 is also connected by way of resistor R5 (X ohms) to output terminal 718 which, by way of the circuitry just described, provides output between 0 and +5 v.d.c. corresponding to a pressure of 0 to 25 cm. water.

A similar output is provided at a corresponding terminal 720 if a second pressure transducer is used. In the dual-conduit embodiment, two transducers provide additional pressure information which allows more precise tracking of inhalation and exhalation gas flows of the patient, and thereby more precise breath cycle tracking.

Fig. 8 is an electrical schematic diagram of microcontroller circuit 800 which includes microcontroller 802 (Intel Type 8097BH), programmable array logic (PAL) (Type PC16L8), erasable, programmable, read-only-memory (EPROM) (Type 27256), address latch 808 (Type 74HC373), random access memory (RAM) (Type 6264P), input/output serial data interface (RS232 Type MAX232), prescription (RX) switch array 814, and input data latch 816.

Microcontroller 802 receives power (Vcc) at +5 v.d.c. at terminals VCC, VPD, BW, RDY, VPP, and VREF as shown. Ground is connected to terminals NMI, VSS, EA, and ANGND. Crystal 802 is coupled between terminals XTAL1 and XTAL2 as shown and to which respective grounded capacitors C1 and C2 (33 pF each) are respectively coupled for timing signals at 12 MHZ.

Microcontroller 802 receives a reset signal at terminal RESET from reset subcircuit 820. On power up, power is supplied through resistor R5 (100K ohms) to grounded capacitor C3 (22 uF) and to the input terminals of SCHMITT trigger NAND gate 822. Initially, the resultant input voltage to NAND 822 is low, and its output is logic high. This logic high output is supplied to output terminal 824 which provides a reset signal to blower motor circuit 900 as discussed further hereinbelow. The initially logic high output from NAND 822 is inverted by invertor 826 to provide a logic low signal to microcontroller terminal RESET which holds microcontroller 802 in reset until the charge on capacitor C3 builds to the trigger level of NAND 822. This provides time for the system to initialize and for transients to be suppressed. As the charge on passer C3 increases to the trigger level, the reset signal is removed from output terminal 824 and microcontroller 802. The output from invertor 826 is also connected to one side of pull-up resistor R6 (10K ohms) the other side of which is connected to Vcc.

Reset circuit 820 also includes a normally open, reset switch 828 coupled across capacitor C3 which allows manual reset. Diode D1 is coupled access resistor R5 to provide a discharge path for C5 in the event of power off.

Microcontroller 802 also receives a pressure transducer input at terminal ACH0 and also at ACH1 if a second transducer is used as in the dual-conduit embodiment. To provide transient suppression, and to smooth the analog voltage from pressure transducer circuit 700, one side of capacitor C4 (.005 nF) is connected to terminal 718 along with the anode of diode D2 and the cathode of diode D3. The other side of capacitor C4 and the anode of diode D3 are connected to ground as shown and the cathode of diode D2 is connected to a supply voltage Vcc. An identical circuit is provided for terminal 720 using diodes D4, D5 and capacitor C5. Microcontroller 802 includes internal analog-to-digital converters (ADC) which receive the respective analog inputs at terminals ACH0 and ACH1 and convert these to digital form for internal use in microcontroller 802.

Microcontroller 802 also receives an input at terminal HS1.0 which is a pulse signal from blower motor circuit 900 representative of the speed of blower unit 18, discussed further hereinbelow.

Microcontroller 802 also uses a common address/data bus 830 which interconnects microcontroller 802 for data and address information flow with PAL 804, EPROM 806, address latch 808, RAM 810, and data latch 816 at the terminals as shown in Fig. 8. Fig. 8 also illustrates the other conventional interconnections between these components as shown.

Microcontroller 802 provides a serial data output from terminal TXD to terminal 11 of interface 812 and receives data from terminal 12 thereof at microcontroller terminal RXD. Interface terminals 14 and 13 receive RS232 data in and out which enable remote reading and control of microcontroller 802 and thereby apparatus 10. This feature is particularly useful in a sleep laboratory, for example, for adjusting the prescription pressures in order to achieve the optimal therapy.

Switch array 814 includes eight, selectable switches for providing input data representative of the desired prescription set point pressures for inhalation and exhalation. In particular, the top four switches are used to set the prescription inhalation pressure and the bottom four switches for prescription exhalation pressure. With four switches for each set point, sixteen possible settings are available ranging between 3 and 16 cm water for inhalation, and 0 and 14 cm water for exhalation. Data latch 816 is coupled with switch array 814 as shown and latches the prescription data upon receipt of the latch signal from terminal 12 of PAL 804. The prescription data is transmitted over bus 830.

Microcontroller 802 also provides two additional outputs. The first of these is data to stepper motor circuit 1000 by way of six-line output bus 832 from microcontroller terminals P1.0-1.5 to output terminal 834. The second additional output is a pulse-width modulated signal (PWM) to blower motor circuit 900 by way of line 834 and output terminal 836.

Fig. 9 is an electrical schematic diagram representing blower motor circuit 900 which receives the pulse width modulated signal at terminal 836 from microcontroller 802, and also receives an inverted reset signal at terminal 824 from reset circuit 820. Blower motor circuit 900 also provides a pulse output signal at terminal 902 representative of the speed of blower motor 904 to microcontroller 802.

The reset signal received at terminal 824 is connected to terminal 10 of motor driver 906 (Type UC3524A). The pulse width modulated signal from controller 802 at terminal 836 is provided to terminal 2 of driver 906 by way of low pass filter C6 (1.0 uF) and resister R7 (24.9K ohms).

Driver terminal 7 is connected to ground by way of capacitor C7 (.003 uF), and terminal 6 is connected to ground by way of resistor R8 (49.9K ohms). Terminal 8 is connected to ground and terminal 15 receives power supply at +12 v.d.c. Driver terminal 12, 13, and 16 are connected to Vcc at +5 v.d.c.

Motor driver 906 converts the input pulse-width modulated signal at 0-5 v.d.c. to a corresponding output at 0 to +12 v.d.c. at terminals 11 and 14 thereof to programmable array logic (PAL) (Type 16L8) terminal 1. These terminals are also connected to ground by way of resistor R9 (0.5 ohms). PAL 908 produces respective outputs at terminals 19 and 18 as two phases for the stator and rotor of brushless D.C. blower motor 904 (Fasco Corp. Type 70000-S517). The PAL 908 outputs are respective inputs to level converters 910 and 912 (MC14504) which shift the voltage level from +5 to +12 v.d.c. The +12 v.d.c. outputs from level converters 910 and 912 are in turn transmitted to the respective gates of field effect transistors (SENSFET) (Motorola SENSFET Type MTP40N06M) 914 and 916. The respective drain terminals of SENSFETS 914 and 916 are respectively connected to terminals 0A and 0B of blower motor 904 and provide the respective phase inputs to the stator and rotor thereof.

Power at +12 v.d.c. is additionally provided to level converters 910 and 912 and to common power terminal CP of blower motor 904.

The source terminal of each SENSFET 914, 916 is connected to ground as shown.

SENSFETS 914,916 each include an additional pair of outputs an lines 918 and 920 which provide a sampling of the current flow through the respective SENSFETS. These outputs are coupled across resistor R10 (100 ohms) to provide a current path for the current sample, and thereby a voltage representative thereof to terminals 3 and 4 of motor driver 906. Driver 906 is responsive to this input voltage representative of the current flow through blower motor 904 to reduce the duty cycle of the output at terminals 11 and 14 in the event of motor overcurrent.

Blower motor 904 is additionally equipped with Hall effect transducer which is operable to provide a voltage pulse each time a magnetic pole of the motor stator passes thereby. These output pulses represent the speed of motor 904 and are provided at motor terminal HALL by way of line 922 to output terminal 902, and as feedback to motor driver 906. The output pulses representative of motor blower speed at terminal 902 are provided to microcontroller 802 at terminal HS1.0 thereof.

The pulses representative of motor blower speed are converted to a representative voltage before input to motor driver terminals 1 and 9. As shown in Fig. 9, line 922 is connected to one side of capacitor C8 (0.01 uF) the other side of which is connected to one side of resistor R11 (10K ohms), and to the anode of diode D6. The other side of resistor R11 is connected to ground.

The cathode of diode D6 is connected to one side of grounded capacitor C9 (0.1 uF), to grounded resistor R12 (1M ohms) and to one side of resistor R13 (100K ohms). The other side of resistor R13 is connected to one side of capacitor C10 (o.22 uF), to one side of resistor R14 (10M ohms), and to motor driver terminal 1 as input thereto. The other side of capacitor C10 and resistor R14 are connected to driver terminal 9.

This network of components C8-C10, R11-R14, and diode D6 convert the frequency pulses on line 922 to a voltage representative thereof. That is to say, this network acts as a frequency-to-voltage converter owing to the large capacitance of capacitor C9 (0.1 uF) which provides a long time constant. The voltage value provided at motor driver terminals 1 and 9 provides feedback to an internal comparator which compares the voltage to a set point derived from the pulse width modulated signal received at terminal 2.

Fig. 10 illustrates stepper motor circuit 1000 which activates stepper motor 44 to position valve element 46 in accordance with data received from microcontroller 802 at terminal 834 therefrom. Stepper motor 44 is preferably a VEXTA® model available from Oriental Motor Company and is capable of providing one revolution in 400 "steps" and is also capable of half-stepping if needed. As those skilled in the art will appreciate, motor 44 is operable to shift one step upon the imposition of the next sequential voltage step pattern provided as input at terminal 834 over output bus 832. In particular, bus 832 includes six lines, which are pattern data for the driver chip.

The step pattern data is provided to step motor driver chip 1002 (Type S'GS' L298N) at terminals A, B, C, and D respectively from terminals P1.0-1.3 of microcontroller 802. Driver 1002 shifts the input data voltage from +5 v.d.c. to +12 v.d.c. for corresponding output at terminals 2, 3, 13, and 14 which are connected to stepper motor 44 to impose the step pattern thereon at +12 v.d.c. The anodes of diodes D7, 8, 9, and 10 are connected to the respective four output lines of driver 1002, and the cathodes thereof are connected to +12 v.d.c. for voltage pull-up. Correspondingly, the cathodes of diodes D11, 12, 13, and 14 are connected respectively to the output lines, and the respective diode cathodes connected to ground as shown for voltage pull-down.

As shown in Fig. 10, +5 v.d.c. is provided at driver terminal 9, +12 v.d.c. at driver terminal 4, and terminals 1, 8, and 15 are all connected to ground.

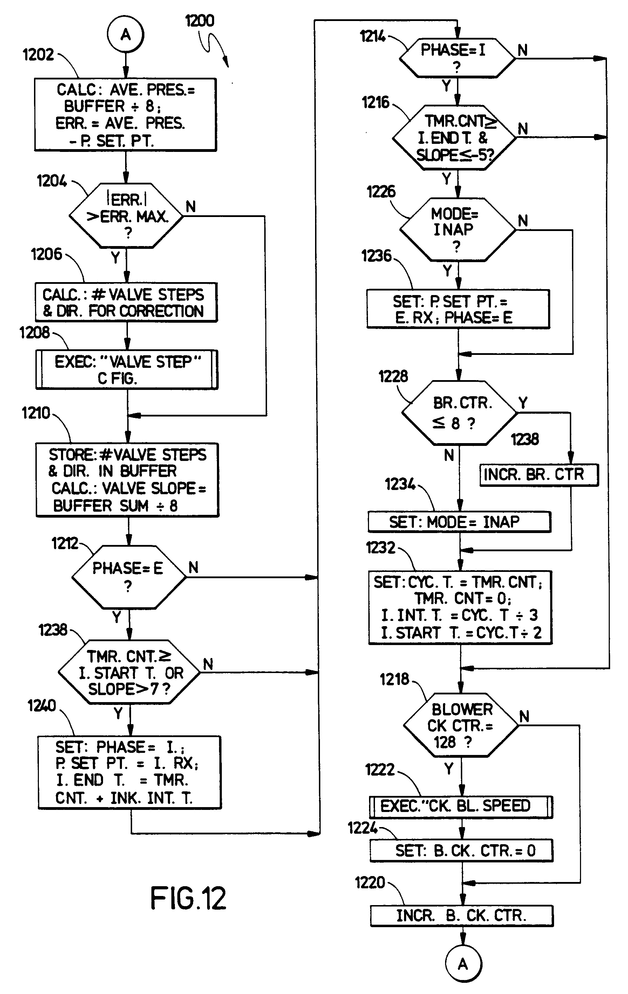

Figs. 11-14 are computer program flowcharts illustrating the operative program for microcontroller 802.

Fig. 11 illustrates the START-UP portion of the main routine of the computer program for operating microcontroller 802. After the logic low reset signal goes logic high, the program enters at step 1102 which prompts controller 20 to shift vent valve assembly 16 to its "home" position. In particular, this step prompts microcontroller 802 to produce data of sequential pattern outputs by way of line 832 and terminal 834 to stepper motor control circuit 1000. This shifts stepper motor 44 to a mid-range position wherein valve element 46 blocks conduit ends 32 and 58 about half-way as shown in Fig. 5, or conduit end 32 alone in the single conduit embodiment. Step 1102 also initializes the variables, counters, interrupt routines, and so forth in the program.

The program then moves to step 1104 to read the inhalation and exhalation prescription pressure values as set on switch array 814 and read by way of address data bus 830. These values are then stored in RAM. Step 1104 also prompts microcontroller 802 to set the operating speed of blower motor 904 in accordance with the prescription of pressure set on switch 814. The blower speed should be set at a level fast enough to ensure that sufficient ambient air volume is provided to conduit 12 such that the prescription pressure level can be attained during maximum inhalation. Blower motor speed data corresponding to prescription settings are stored preferably in a look-up table. Step 1104 also clears any value stored in the internal buffer at microcontroller terminal HS1.0.

The program then moves to step 1106 which enables the program's timed interrupts to begin timing.

In step 1108 the program sets the software flag "phase" equal to inhalation "I" which initializes the program from the inhalation phase of the patient's breathing cycle. This step also initializes the blower check counter at zero. As discussed further hereinbelow, the program reads the blower speed after 128 passes through the main loop.

The program then moves to step 1110 which starts the internal analog-to-digital converter (ADC) connected to microcontroller input terminals ACH0 and ACH1.

Step 1112 sets the pressure set point for the inhalation phase according to the inhalation prescription value set on switch array 814 according to data in a look-up table. This step also defines the start-up mode of the apparatus as continuous positive airway pressure (CPAP). That is to say, and as explained further hereinbelow, the program operates apparatus 10 in order to present a continuous positive pressure at the inhalation set point pressure for the first eight breaths of a patient. Step 1112 also initializes the breath counter at zero in preparation for counting patient breathing cycles.

After completion of step 1112 the program moves to MAIN LOOP 1200 of the main routine as illustrated in Fig. 12. Step 1202 is the first step of this routine in which the program calculates the average pressure as sensed by pressure transducer 701 over eight ADC conversions. `That is to say, microcontroller 802 includes an internal "ring" buffer which stores the eight most recent pressure readings received at microcontroller terminal ACH0 (and also ACH1 in the two-conduit embodiment). As discussed further hereinbelow, ADC interrupt routine converts the input analog values to digital form every 22 microseconds and continuously stores the most recent digital values in the ring buffer. Step 1020 calculates the average value by dividing the cumulative buffer value by eight. Step 1202 also calculates the deviation, that is, error, in the average pressure from the pressure set point.

The program then moves to step 1204 which asks whether the magnitude of the error calculated in step 1202 is greater than allowed maximum error. This provides a so-called "dead band" to prevent the system from "hunting".

If the answer in step 1204 is yes, the program moves to step 1206 and calculates the number of steps and direction of stepper motor 44 required to correct the pressure deviation error. That is to say, depending upon the volume of air being produced by the blower, the fluid capacity of the system, and the leakage therefrom, the number of required steps can be determined approximately by reference to data previously stored in a look-up table.

The program then moves to step 1208 to execute routine "VALVE STEP" illustrated in Fig. 13 and discussed further hereinbelow. VALVE STEP routine 1300 sequentially presents the data patterns required to step the valve for the required number of steps in the direction determined in step 1206.

After execution of sub-routine 1300 or after step 1204, the program returns to step 1210. This step stores the number of valve steps and direction actually implemented in an internal valve slope buffer which continuously stores the previous eight movements of stepper motor 44. With this information, the slope of valve movement can be calculated by dividing the valve slope buffer sum by eight. This represents a slope because the eight values are stored at equal time intervals and thus the buffer sum divided by eight represents the first derivative of value movement.

For example, and referring to Fig. 6, after the post-exhalation pause, and after achieving the desired set point pressure, no significant error in pressure versus set point exists. Thus, no change in the value position is required and so the previous eight value steps would equal zero, indicating a slope of zero, which is indicated by the flat portion of the valve position curve in Fig. 6. In contrast, when the patient begins to inhale, the valve position must initially and quickly shift toward the closed position to maintain the pressure in conduit 32. With a number of positive steps executed on stepper motor 44, the values stored in the slope buffer indicate a high positive slope. Conversely, near the end of inhalation, the valve must execute a number of steps in the negative direction in order to maintain the pressure in conduit 32 indicating a large negative slope. This slope information, as is discussed further hereinbelow, is use to determine various points in the breathing cycle of a patient.

The program then moves to step 1212 which asks whether the phase flag is set for exhalation. The program was initialized with the phase flag set for inhalation, and so, during the first few passes through main loop 1200, the answer in 1212 is no and the program moves to step 1214 which asks whether the phase flag is set for inhalation. Because this flag is initialized as inhalation, the answer in step 1214 is yes and the program moves to step 1216.

Step 1216 asks whether the variable "timer counter" is greater than the value for variable "inhalation end time", and whether the slope as calculated in step 1210 is less than or equal to -5. The variable "timer counter" (TMR CNT) is a software counter which was initialized at zero and increments every 13 milliseconds. The variable "inhalation end time" was initialized at a default value representing inhalation time equivalent to a predetermined average value. As discussed further hereinbelow, the variable "inhalation end time" is recalculated for each breath cycle after an initial eight passes through main loop 1200. Step 1216 operates to determine whether sufficient time has passed for normal inhalation to be complete as additionally confirmed by the value slope being less than -5 as illustrated by the slope of the value position curve at the end of inhalation in Fig. 6.

During the first few passes through main loop 1200, the answer in step 1216 is no and the program moves to step 1218 which asks whether the blower check counter, initialized at zero, is equal to 128. Until then, the answer in step 1218 is no and the program moves to step 1220 to increment the blower check counter. The program then loops back to step 1202 and repetitively executes steps 1202-1220 until the answer in step 1218 is yes whereupon the program moves to step 1222 to execute the sub-routine "CHECK BLOWER SPEED" 1200 as illustrated in Fig. 15. As discussed further hereinbelow, this step monitors the blower speed to ensure that it is running at the set point speed initially set in step 1104 in accordance with prescription settings. The program then returns to step 1224 to reset the blower check counter at zero.

After sufficient time has elapsed to exceed the default time set for the inhalation end time, and when the slope of the valve position curve is equal to or less than -5 indicating the end of patient inhalation, the answer in step 1216 is yes and the program moves to step 1218 which asks whether the mode of operation is set for inspiratory nasal air pressure (INAP). This was initialized in the CPAP mode in step 1112. During the first eight breathing cycle, the answer in step 1226 is no, and the program moves to step 1228 which asks whether the breath counter is less than or equal to eight. The breath counter was initialized at zero and during the first pass of the program the answer in step 1220 is yes, and the program moves to step 1230 to increment the breath counter.

The program then moves to step 1232 which sets the variable "cycle time" equal to the current value existing on the timer counter. This step is entered at the end of each inhalation phase and marks the end of one breath cycle and the beginning of another. Thus, the time of one breath cycle, that is, cycle time, equals the time value existing on the timer counter which is reset to zero at the end of each breath cycle, also in step 1232.

Step 1232 also sets a new inhalation interval time equal to the new cycle time divided by three. Statistically, inhalation time averages about 40% of a typical breathing cycle. Step 1232, however, sets the inhalation interval equal to 33% of the most recent cycle time in order to ensure that this value clocks out in step 1216 early, that is, before the end of anticipated actual inhalation time.

Step 1232 also sets the variable "inhalation start time" equal to the new cycle time divided by two. With the beginning of a cycle marked as the end of an inhalation phase, the next inhalation start time would normally be expected to occur after 60% of the cycle time has elapsed. Step 1232, however, sets inhalation start time at 50%, that is earlier than the predicted inhalation time in order to ensure an increase in nasal pressure before inhalation would be expected to begin.

After main loop 1200 has detected eight breath cycles as indicated on the breath counter, the answer in step 1228 is no and the program moves to step 1234 which sets the operating mode as INAP. The eight cycle delay in setting the INAP mode ensures reliable data in tracking the breath cycle.

With the mode now set as INAP, the answer during the next pass at step 1226 is yes and the program moves to step 1236 to set the pressure set point equal to the exhaust prescription. That is to say, an inhalation phase has ended as determined in step 1216, eight breaths have been tracked as determined in step 1228, the mode is set as INAP which allows a decrease in pressure during exhalation. With these conditions satisfied, the controlled pressure set point is lowered to the prescribed exhaust prescription set point.

Normally, the exhaust pressure would be prescribed at zero, that is ambient, so that the patient can exhale normally. In some circumstances, however, the therapist may desire a slight positive pressure during exhalation which is set on the lower four switches of switch array 814 (Fig. 8).

Step 1236 also sets the phase flag for exhalation.

During the next pass through main loop 1200, the answer in step 1212 is now yes, that is, the phase is "exhalation", and the program moves to step 1238 which asks whether the current value on the timer counter is greater than or equal to the inha[ation start time as previously set in step 1232. In the alternative, step 1238 asks whether the valve position slope is greater than seven which independently indicates the end of exhalation. With reference to Fig. 6, at the end of exhalation, the valve must step in the positive direction rapidly in order to restrict vent end 32 for maintaining the set point pressure. This rapid change indicates a positive slope greater than 70.

If the answer in step 1238 is no, the program continues to loop through until the answer is yes at which time the program moves to step 1240 to set the phase flag for inhalation, to set the pressure set point at the inhalation prescription value, and to set the value for the variable "inhalation end time" equal to the currently existing timer count plus the inhalation interval time. The existing value of the timer counter corresponds to the time elapsed since the beginning of the current breath cycle, which marked the end of the previous inhalation phase. The inhalation phase about to begin should end on or after the current timer count value plus the inhalation interval time. Thus, step 1240 provides a new value for inhalation interval time for use in step 1216. Normally, this value is reached before the end of the actual inhalation and is used to ensure that a transient slope reading does not erroneously mark the end of the inhalation phase. Thus the requirement in step 1216 for both the expiration of the inhalation end time and a slope less than or equal to -5.

As those skilled in the art will appreciate, step 1238, in cooperation with the balance of the operating program, ensures that the inhalation set point pressure increases before the onset of patient inhalation. First, by monitoring whether the valve position slope exceeds seven, the end of exhalation can be detected. Marking the end of an exhalation phase ensures that this is a point in the breath cycle prior to the beginning of the next inhalation phase. Additionally, an increase in the pressure prior to inhalation is assured by monitoring whether the timer counter is greater than or equal to the predicted inhalation start time in step 1238. Thus, if a sporadic or erroneous slope reading were determined, an increase in nasal pressure would still be ensured prior to inhalation when the timer counter excess the predicted inhalation start time, recalling that the inhalation start time was set in step 1232 somewhat shorter than the expected start time.

Fig. 13 illustrates VALVE STEP sub-routine 1300 which operates to impose sequentially the required step patterns on stepper motor 44 by way of stepper motor circuit 1000. Sub-routine 1300 enters at step 1302 by setting the variable "final valve position" equal to the current valve position plus (or minus) the valve correction required as determined in step 1206 (Fig. 2). Step 1302 also sets the variable "valve position" equal to the current valve position.

The program then moves to step 1304 which asks whether the correction direction is greater than zero, that is, in a positive direction to restrict vent end 32, or in the opposite direction. If the answer in step 1304 is yes, the program moves to step 1306 which asks whether the final position as determined in step 1302 exceeds step 160. That is to say, this step determines whether the requested or desired final valve position is beyond the maximum allowed position. If yes, the program moves to step 1308 which sets the final valve position equal to 160.

If the answer in step 1306 is no, or after step 1308, the program moves to step 1310 to set the variable "valve position" equal to "valve position" plus one. In other words, the program increments stepper motor 44 one step at a time until the final position is achieved.

The program then moves to step 1312 which asks whether the new valve position is less than or equal to the final valve position as determined in step 1302. If no, which indicates that the desired final valve position has been achieved, the program returns to main loop step 1210.

If the answer in step 1312 is yes, indicating that the final valve position has not yet been achieved the program moves to step 1314 which retrieves the step pattern for the next blower motor step from memory. The program then activates the lines of bus 832 in order to send this step pattern to stepper motor circuit 1000 and thereby to stepper motor 34.

The program then loops back to step 1310 to continue executing step patterns one at a time in sequence until the final position is obtained.

If the rotational direction for correction requires is negative as determined in step 1304, the program moves to steps 1316-1324 as illustrated to execute the required number of stepping patterns to shift the valve in the "negative' direction to reduce pressure by venting more air. Step 1316 asks whether the final position determined in step 1302 is less than zero indicating a valve position beyond the allowable limits of travel. If yes, the program sets the final position equal to zero in step 1318.

Step 1320 then decrements the "valve position" variable and step 1322 asks whether the newly determined "valve position" is greater than or equal to the final position desired. If yes, the step moves to program 1324 and then loops back to step 1322. If the answer is step 1322 is no, the program returns to main loop step 1210.

Fig. 14 illustrates ADC interrupt sub-routine 1400 which has its interrupt executed every X micro-seconds for providing an analog-to-digital conversion for the pressure data received from pressure transducer circuit 700, and to store this data in memory. Subroutine 1400 enters at step 1402 which retrieves the current data from the ADC register internal to microcontroller 802. This data is then stored in the ADC buffer for use in step 1202 (Fig. 12) of the main loop. This data is stored at location "L" which is one of the eight buffer locations. The program then moves to step 1404 to increment location variable "L" so that the next set of ADC data is placed in the next buffer location. The program then moves to step 1406 which asks whether "L" is equal to eight which is greater than the number of locations provided in the ADC buffer. If yes, the program resets "L" at location zero which is the first location in the buffer. After step 1408, or if the answer in step 1406 is no, the program moves to step 1410 which instructs the ADC to begin another data conversion. The program then returns from the interrupt to the main loop.

Fig. 15 illustrates CHECK BLOWER SPEED subroutine 1500 which is entered from step 1222 of main loop 1200, and enters at step 1502 which reads the current blower speed as received at microcontroller terminal HS1.0 from the Hall effect transducer in blower motor 94. The program then moves to step 1504 which retrieves the blower speed set point corresponding to the prescription inhalation pressure and compares the set point to the sensed lower speed. The program then moves to step 1506 which asks whether the blower speed is within a maximum error range of the set point speed. If no, the program adjusts, in step 1508, the pulse-width of the pulse width modulated signal produced at microcontroller terminal PWM and transmitted to blower motor circuit 900. After step 1508, or if the answer in step 1506 is yes, the program returns to the main loop.

As those skilled in the art will appreciate, the present invention encompasses many variations in the preferred embodiments described herein. For example while the present invention is useful in treating sleep apnea, its utility is not so limited, but rather, the present invention is useful in treating many conditions in which facilitated respiration is a factor in treatment. For example, increased respiratory air pressure beginning just prior to inhalation induces a deeper inhalation than might otherwise occur. This may be useful in treating certain cardiovascular conditions where deeper inhalation and thereby greater oxygenation of the blood is beneficial when accompanied by decreased pressure to ease exhalation. Additionally, the present invention encompasses the use of any breathable gas such as anesthesia or oxygen-supplemented ambient air.

As discussed above, the nasal pillow is the preferred means for patient coupling in order to impose the higher breathable gas pressure on the respiratory passages of the patient. The present invention, however, also encompasses a nasal mask, or a full face mask which may be desired in certain situations such as the application of anesthesia as breathable gas as discussed above.

In the preferred embodiment of the present invention, the position of the vent valve assembly is varied in order to increase or decrease the pressure of the breathable gas applied to the patient's respiratory passages. As the detailed description reveals, however, the apparatus hereof includes the capability of varying the speed of the blower unit which could be used instead to selectively vary the applied pressure. This would eliminate the need for the vent valve and stepper motor and reduce the manufacturing cost which would be advantageous as another embodiment of the invention.

The present invention also encompasses the variation wherein the breathable gas is compressed and stored in a storage bottle, for example.

As described above, the preferred controller includes microcontroller 802 which is operated by a computer program. Other equivalent control means might include a custom designed chip with all functions implemented in hardware without a computer program.

As disclosed in Fig. 6, it is preferred to track the patient's breathing cycle by tracking the movement of vent valve assembly 16. However, the breath cycle can be tracked by other means such as monitoring chest contractions and expansion, breathing sounds, directly sensing genioglossus muscle activity, or some equivalent parameter indicative of a breathing cycle.

As a final example, some therapists may prefer that the apparatus start up in a low pressure or zero pressure mode while the breath cycle is initially tracked. This may provide further patient comfort in the use of the invention.

## Claims

1. Continuous positive airway pressure breathing apparatus (10) for treating sleep apnea, which comprises:
(a) gas supply means (12, 18) for providing a breathing gas at a positive pressure of a magnitude at least equal to ambient atmospheric pressure to the airway of a patient continuously through the patient's breathing cycle,
(b) means (20) for controlling the supply of the breathing gas,
characterised in that the gas supply control means (20) tracks the spontaneous breathing of a patient to determine the occurrence of the inspiratory and expiratory phases of the breathing cycle, and coordinates the magnitude of the positive pressure of breathing gas with the breathing pressure which occurs during the inspiratory and expiratory phases of the patient's breathing cycle, the gas supply control means comprising a pressure transducer (700), storage means for pressure variances detected by the pressure transducer, and means (16) for regulating the supply of gas in response to signals from the pressure transducer so that the magnitude of the positive pressure maintained during the expiratory phase is less than that during the immediately preceding inspiratory phase.

2. Apparatus as claimed in claim 1, in which the gas supply means comprises a blower (18) whose speed can be adjusted, and in which the gas supply control means (20) is capable of adjusting the speed of the blower to change the pressure of gas supplied to the patient.

3. Apparatus as claimed in claim 1, which includes a valve (16) which cooperates with the gas supply control means (20) for selectively venting breathing gas to change the pressure of gas supplied to the patient.

4. Apparatus as claimed in claim 3, in which the means for providing breathing gas includes a blower (18).

5. Apparatus as claimed in claim 4, in which the means for providing breathing gas includes a conduit (12) coupled to the blower (18), which can be connected to the airway of the patient.

6. Apparatus as claimed in claim 5, in which the valve (16) comprises a vent valve in fluid communication with the blower (18) and the conduit (12).

7. Apparatus as claimed in claim 5, in which the conduit (12) includes a delivery conduit (60) for delivering gas from the gas supply means to the patient, and an exhaust conduit (52) for conveying exhaled gas from the patient to the valve (16).

8. Apparatus as claimed in claim 7, in which the delivery and exhaust conduits (52, 60) each include a check valve (62, 64) for directionally limiting gas flow therethrough.

9. Apparatus as claimed in claim 1, in which the gas supply control means (20) includes means for determining the rate of flow of the gas and direction of the gas flow inhaled and exhaled by the patient during the breathing cycle to provide an indication of the inhalation and exhalation phases of the breathing cycle.

10. Apparatus as claimed in claim 1, in which the gas supply control means (20) includes means for determining a predicted inhalation start time correlated with at least one prior breathing cycle, and for initiating a gas pressure increase at a point in the breathing cycle correlated therewith.

11. Apparatus as claimed in claim 6, in which the gas supply control means (20) communicates with the vent valve (16) to maintain a set point pressure of the breathing gas supplied to the patient.

12. Apparatus as claimed in any one of claims 1 to 11, which includes a device (14) for coupling breathing gas to the nares of the patient.

## Patentansprüche

1. Atemgerät (10) mit kontinuierlichem positivem Druck im Luftweg zur Behandlung einer Schlafapnoe, welches umfaßt:
(a) eine Gasversorgungseinrichtung (12,18), mit der ein Atemgas bei einem positiven Druck einer Größe, welche mindestens gleich dem Atmosphärendruck in der Umgebung ist, dem Luftweg des Patienten während des Atemzyklus des Patienten kontinuierlich bereitstellbar ist;
(b) eine Einrichtung (20) mit der die Zufuhr des Atemgases gesteuert wird,
dadurch gekennzeichnet, daß die Steuereinrichtung (20) für die Gaszufuhr die spontane Atmung des Patienten verfolgt, so daß das Auftreten der Phasen des Einatmens und des Ausatmens des Atemzyklus festgestellt wird, und die Größe des positiven Druckes des Atemgases auf den Atemdruck abstimmt, welcher während der Phasen des Einatmens und des Ausatmens des Atemzyklus des Patienten vorhanden ist, wobei die Steuereinrichtung für die Gaszufuhr einen Druckwandler (700), Speichermittel für Druckänderungen, welche von dem Druckwandler erfaßt werden, und Mittel (16) umfaßt, mit denen die Gaszufuhr geregelt wird und welche hierzu auf Signale des Druckwandlers so ansprechen, daß die Größe des positiven Druckes, welcher während der Phase des Ausatmens aufrechterhalten wird, geringer ist als jene während der unmittelbar vorhergehenden Phase des Einatmens.

2. Gerät nach Anspruch 1, bei dem die Gasversorgungseinrichtung ein Gebläse (18) umfaßt, dessen Drehzahl eingestellt werden kann, und bei dem die Steuereinrichtung (20) für die Gaszufuhr die Drehzahl des Gebläses einstellen kann, um den Druck des Gases, welches dem Patienten zugeführt wird, zu ändern.

3. Gerät nach Anspruch 1, welches ein Ventil (16) umfaßt, das mit der Steuereinrichtung (20) für die Gaszufuhr zusammenarbeitet und mit dem wahlweise Atemgas abgelassen wird, um den Druck des Gases, welches dem Patienten zugeführt wird, zu ändern.

4. Gerät nach Anspruch 3, bei dem die Mittel, mit denen Atemgas bereitgestellt wird, ein Gebläse (18) umfassen.

5. Gerät nach Anspruch 4, bei dem die Mittel, mit denen Atemgas bereitgestellt wird, eine Leitung (12) umfassen, welche mit dem Gebläse (18) gekoppelt ist und welche mit dem Luftweg des Patienten verbunden werden kann.

6. Gerät nach Anspruch 5, bei dem das Ventil (16) ein Entlüftungsventil umfaßt, welches in Fluidverbindung mit dem Gebläse (18) und der Leitung (12) steht.

7. Gerät nach Anspruch 5, bei dem die Leitung (12) eine Förderleitung (60), mit der Gas von der Gasversorgungseinrichtung zu dem Patienten geführt wird, und eine Auslaßleitung (52) umfaßt, mit der ausgeatmetes Gas von dem Patienten zu dem Ventil (16) geführt wird.

8. Gerät nach Anspruch 7, bei dem die Förderleitung (60) und die Auslaßleitung (52) jeweils ein Rückschlagventil (62, 64) aufweisen, mit dem die durch sie hindurchfließende Gasströmung in einer Richtung begrenzt wird.

9. Gerät nach Anspruch 1, bei dem die Steuereinrichtung (20) für die Gaszufuhr Mittel umfaßt, mit denen der Gasdurchsatz und die Richtung der Strömung des Gases bestimmt werden, welches während des Atemzyklus von dem Patienten eingeatmet und ausgeatmet wird, so daß eine Anzeige der Phasen des Einatmens und des Ausatmens des Atemzyklus geschaffen wird.

10. Gerät nach Anspruch 1, bei dem die Steuereinrichtung (20) für die Gaszufuhr Mittel umfaßt, mit denen eine voraussichtliche Startzeit für das Einatmen bestimmt wird, welche mit mindestens einem vorhergehenden Atemzyklus korreliert ist, und mit denen eine Erhöhung des Gasdruckes an einem Punkt im Atemzyklus initiiert wird, welcher damit korreliert ist.

11. Gerät nach Anspruch 6, bei dem die Steuereinrichtung (20) für die Gasversorgung mit dem Entlüftungsventil (16) kommuniziert und so einen Soll-Druck des Atemgases aufrechterhält, welches dem Patienten zugeführt wird.

12. Gerät nach einem der Ansprüche 1 bis 11, welches eine Vorrichtung (14) umfaßt, mit der Atemgas an die Nasenlöchern des Patienten koppelbar ist.

## Revendications

1. Appareil respiratoire (10) exerçant une pression positive continue sur les voies aériennes pour traiter l'apnée pendant le sommeil, qui comprend :
(a) des moyens (12, 18) d'alimentation en gaz pour fournir un gaz respiratoire à une pression positive d'une ampleur au moins égale à la pression atmosphérique ambiante aux voies aériennes d'un patient continûment pendant le cycle de respiration du patient,
(b) des moyens (20) pour commander l'alimentation en gaz respiratoire,
caractérisé en ce que les moyens (20) de commande d'alimentation en gaz suivent la respiration spontanée d'un patient pour déterminer l'occurrence des temps d'inspiration et d'expiration du cycle de respiration, et coordonnent l'ampleur de la pression positive du gaz respiratoire avec la pression respiratoire qui se produit pendant les temps d'inspiration et d'expiration du cycle de respiration du patient, les moyens de commande d'alimentation en gaz comprenant un capteur de pression (700), des moyens de stockage des variations de pression détectées par le capteur de pression, et des moyens (16) pour régler l'alimentation en gaz en réponse à des signaux provenant du capteur de pression de sorte que l'ampleur de la pression positive maintenue pendant le temps d'expiration est inférieure à celle maintenue pendant le temps d'inspiration précédent immédiatement celui-ci.

2. Appareil selon la revendication 1, dans lequel les moyens d'alimentation en gaz comprennent une soufflante (18) dont la vitesse peut être réglée, et dans lequel les moyens (20) de commande d'alimentation en gaz sont capables de régler la vitesse de la soufflante pour modifier la pression de gaz fourni au patient.

3. Appareil selon la revendication 1, comprenant une vanne (16) qui coopère avec les moyens (20) de commande d'alimentation en gaz pour décharger sélectivement le gaz respiratoire pour modifier la pression de gaz fourni au patient.

4. Appareil selon la revendication 3, dans lequel les moyens pour fournir le gaz respiratoire comprennent une soufflante (18).

5. Appareil selon la revendication 4, dans lequel les moyens pour fournir le gaz respiratoire comprennent un conduit (12) accouplé à la soufflante (18), qui peut être raccordé aux voies aériennes du patient.

6. Appareil selon la revendication 5, dans lequel la vanne (16) comprend une vanne de décharge en communication de fluide avec la soufflante (18) et le conduit (12).

7. Appareil selon la revendication 5, dans lequel le conduit (12) comporte un conduit de fourniture (60) pour fournir le gaz provenant des moyens d'alimentation en gaz au patient, et un conduit d'échappement (52) pour transporter le gaz expiré depuis le patient jusqu'à la vanne (16).

8. Appareil selon la revendication 7, dans lequel les conduits de fourniture et d'échappement (52, 60) comprennent chacun un clapet anti-retour (62, 64) pour limiter de manière directionnelle le flux de gaz les traversant.

9. Appareil selon la revendication 1, dans lequel les moyens (20) de commande d'alimentation en gaz comprennent des moyens pour déterminer le débit de gaz et la direction du flux de gaz inspiré et expiré par le patient pendant le cycle de respiration pour fournir une indication des temps d'inspiration et d'expiration du cycle de respiration.

10. Appareil selon la revendication 1, dans lequel les moyens (20) de commande d'alimentation en gaz comprennent des moyens pour déterminer un moment de début d'inspiration prévu en corrélation avec au moins un cycle de respiration précédent, et pour initier une augmentation de pression de gaz au niveau d'un point dans le cycle de respiration en corrélation avec celui-ci.

11. Appareil selon la revendication 6, dans lequel les moyens (20) de commande d'alimentation en gaz communiquent avec la vanne de décharge (16) pour maintenir une pression de consigne du gaz respiratoire fourni au patient.

12. Appareil selon l'une quelconque des revendications 1 à 11, comprenant un dispositif (14) pour relier le gaz respiratoire aux narines du patient.
